# EUROPÄISCHE PATENTSCHRIFT

(11) **EP 0 899 857 B1**
(45) Veröffentlichungstag und Bekanntmachung des Hinweises auf die Patenterteilung: **28.11.2007**
(21) Anmeldenummer: 97810595.5
(22) Anmeldetag: 25.08.1997
(51) Int. Cl.: H02K 49/06

(54) **Magnetgelagerte Rotationsanordnung**
Rotary device with magnetic bearing
Dispositif rotatif avec palier magnétique

(43) Veröffentlichungstag der Anmeldung: 03.03.1999
(73) Patentinhaber: Lust Antriebstechnik GmbH, D-35633 Lahnau (DE)
(72) Erfinder: Schöb, Reto, Dr., CH-8604 Volketswil (DE)
(74) Vertreter: Sulzer Management AG

(56) Entgegenhaltungen:
- EP-A- 0 130 541
- EP-A- 0 501 427
- WO-A-96/19034
- WO-A-96/31934
- WO-A-97/15978
- FR-A- 1 464 894
- US-A- 5 350 283

## Beschreibung

Die Erfindung betrifft eine magnetgelagerte Rotationsanordnung gemäss dem Oberbegriff von Patentanspruch 1. Ferner betrifft die Erfindung Fördervorrichtungen, Trägervorrichtungen, Gebläse sowie Rührwerke mit einer derartigen Rotationsanordnung.

Magnetgelagerte Rotationsanordnungen kommen heutzutage in vielen Anwendungsgebieten zum Einsatz, insbesondere aber dort, wo mechanisch gelagerte Rotationsanordnungen erhebliche Nachteile aufweisen, so z.B. bei Rührwerken oder Fördervorrichtungen für hochreine oder biologische Flüssigkeiten wie beispielsweise Blut. Auch bei Anwendungen wie Reinraumgebläsen, wo hohe Anforderungen an die Reinheit gestellt werden und es zu keinerlei Verunreinigungen kommen darf, wie sie z.B. durch Gase hervorgerufen werden können, die aus Schmiermitteln von mechanischen Lagerungen entweichen können, kommen magnetgelagerte Rotationsanordnungen zum Einsatz. Bei diesen Anwendungen muss sowohl eine Lagerung des Rotors (der z.B. als Flügelrad einer Pumpe ausgebildet sein kann) als auch ein rotatorische Bewegung desselben möglich sein.

In der US-A-5,350,283 wird eine Pumpe vorgeschlagen, in der eine magnetgelagerte Rotationsanordnung zum Einsatz kommt. Die dort beschriebene Pumpe weist einen im Innenraum der Pumpe angeordnetes Laufrad (z.B. ein Flügelrad) auf, das in axialer Richtung magnetisch gelagert ist. Das Laufrad weist zu diesem Zweck auf seiner einen Seite in axialer Richtung weisende Permanentmagnete auf. Ausserhalb des Pumpengehäuses ist ein Rotor angeordnet, der ebenfalls mit entsprechend angeordneten Permanentmagneten versehen ist. Die Permanentmagnete des ausserhalb des Pumpengehäuses angeordneten Rotors sind ebenfalls in axialer Richtung weisend angeordnet und zwar so, dass sie gegenüber den Permanentmagneten des Rotors zu liegen kommen. Auf der anderen Seite ist das Laufrad mit U-förmigem Weicheisen belegt. Ausserhalb des Pumpengehäuses, gegenüber dem U-förmigen Weicheisen, sind entsprechende U-förmige Steuermagnete angeordnet, die die eigentliche Lagerung des Laufrads bewirken. Die Steuermagnete weisen einen Permanentmagneten zur Erzeugung einer Vormagnetisierung auf sowie Steuerwicklungen, sie sind ortsfest am Pumpengehäuse angeordnet.

WO96/19034 A offenbart eine Rotationsanordnung gemäß dem Oberbegriff des Anspruchs 1.

Aufgabe der Erfindung ist es, eine möglichst wenig aufwendige magnetgelagerte Rotationsanordnung vorzuschlagen, mit welcher gleichzeitig eine magnetische Lagerung wie auch eine Drehung des Rotors bewirkt werden kann. Die magnetgelagerte Rotationsanordnung soll dabei auch die eingangs genannten Anwendungen gestatten können.

Erfindungsgemäss wird diese Aufgabe durch eine Rotationsanordnung gelöst wie sie durch die Merkmale des Patentanspruchs 1 charakterisiert ist. Vorteilhafte Ausgestaltungen ergeben sich aus den abhängigen Patentansprüchen.

Im folgenden wird die Erfindung anhand der Zeichnung näher erläutert. Es zeigen schematisch und/oder im Schnitt:
- Fig. 1: eine Prinzipskizze eines Ausführungsbeispiels einer erfindungemässen Rotationsanordnung zur Erläuterung des Funktionsprinzips,
- Fig. 2: ein Ausführungsbeispiel einer erfindungsgemässen Rotationsanordnung,
- Fig. 3: ein Ausführungsbeispiel des Lagerstators und des Rotors,
- Fig. 4: ein weiteres Ausführungsbeispiel des Lagerstators und des Rotors,
- Fig. 5: verschiedene Kombinationen der Anordnung von Permanentmagneten auf dem Lagerstator und dem Rotor,
- Fig. 6: einen Ausschnitt von einander gegenüberliegenden Zähnen von Lagerstator und Rotor mit Nuten auf den einander zugewandten Flächen,
- Fig. 7: ein weiteres Ausführungsbeispiel einer erfindungsgemässen Rotationsanordnung,
- Fig. 8: eine Prinzipskizze einer Regelung der Position des Rotors,
- Fig. 9: ein weiteres Ausführungsbeispiel einer erfindungsgemässen Rotationsanordnung,
- Fig. 10: einen Schnitt entlang der Linie X-X in Fig. 9,
- Fig. 11: einen Schnitt entlang der Linie XI-XI in Fig. 9,
- Fig. 12: ein weiteres Ausführungsbeispiel einer erfindungsgemässen Rotationsanordnung, mit Permanentmagneten im Rotor und Steuerwicklungen in beiden Statorringen,
- Fig. 13: ein weiteres Ausführungsbeispiel einer erfindungsgemässen Rotationsanordnung, mit Permantenmagneten im Rotor und Steuerwicklungen nur in einem Statorring,
- Fig. 14: einen sternförmigen Rotor mit vier Ästen,
- Fig. 15: einen sternförmigen Stator mit U-förmigen Spulenkernen für einen Rotor gemäss Fig. 14,
- Fig. 16: einen sternförmigen Rotor mit drei Ästen und Permanentmagneten,
- Fig. 17: einen sternförmigen Stator mit drei Ästen und mit U-förmigen Spulenkernen,
- Fig. 18: einen sternförmigen Rotor mit vier Ästen und Permantenmagneten,
- Fig. 19: einen sternförmigen Stator mit vier Ästen, mit U-förmigen Spulenkernen und mit einer Ringwicklung,
- Fig. 20: einen sternförmigen Stator mit vier Ästen, mit U-förmigen Spulenkeren und mit vier separaten Steuerwicklungen,
- Fig. 21: ein Ausführungsbeispiel einer Anwendung einer erfindungsgemässen Rotationsanordnung mit einer radialen Magnetlagerung,
- Fig. 22: ein Ausführungsbeispiel einer Anwendung einer erfindungsgemässen Rotationsanordnung mit einer axialen Magnetlagerung
- Fig. 23: ein weiteres Ausführungsbeispiel einer Anwendung einer erfindungsgemässen Rotationsanordnung in Form einer Pumpe
und
- Fig. 24: das Ausführungsbeispiel gemäss Fig. 4 mit Hall-Sensoren.

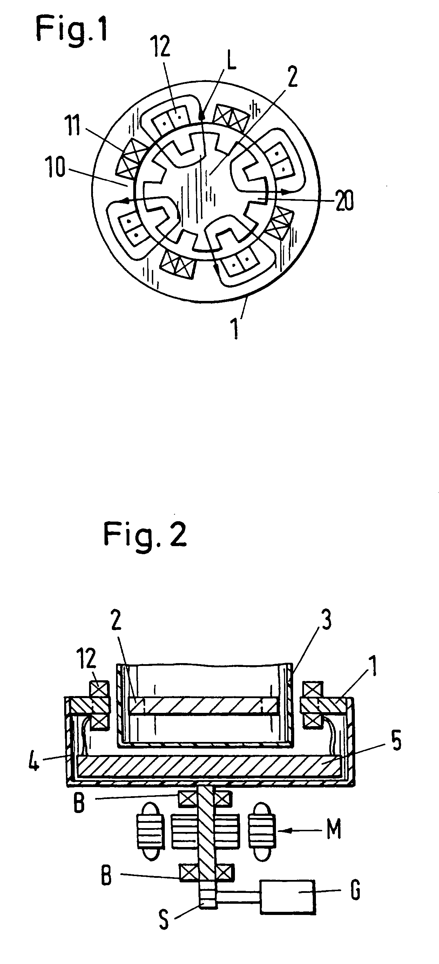

In der in Fig. 1 dargestellten Prinzipskizze eines Ausführungsbeispiels der erfindungsgemässen Rotationsanordnung (hier ein Radialmagnetlager) erkennt man einen Lagerstator 1 und einen Rotor 2. Der Lagerstator 1 weist Zähne 10 auf und der Rotor 2 entsprechende Zähne 20, wobei Zähne 10 und 20 des Lagerstators 1 und des Rotors 2 aufeinander zuweisend angeordnet sind. Die Anzahl der Zähne 10 des Lagerstators 1 und der Zähne 20 des Rotors 2 stimmen überein. Zwischen den Zähnen 10 des Lagerstators 1 befinden sich Nuten 11, zwischen den Zähnen 20 des Rotors 2 befinden sich entsprechende Nuten 21. Um die einzelnen Zähne 10 des Lagerstators 1 herum sind Wicklungen 12 gewickelt. Im Betrieb sind diese Wicklungen z.B. in der dargestellten Weise stromdurchflossen, so dass die durch die Pfeile L angedeuteten magnetischen Kreise gebildet werden. Der besseren Übersichtlichkeit halber sind in Fig. 1 nur einige dieser magnetischen Kreise repräsentativ angedeutet.

Der Rotor 2 wird in der Sollage in radialer Richtung gleichmässig nach allen Seiten vom Lagerstator 1 angezogen, wodurch die magnetische Lagerung des Rotors 2 bewirkt wird. Lagerstator 1 und Rotor 2 sind also magnetisch gekoppelt. Wird nun der Lagerstator 1 gedreht, so dreht sich aufgrund von Reluktanzkräften (die Reluktanzkräfte wirken stets so, dass die Zähne bestrebt sind, einander gegenüber zu liegen) auch der Rotor 2 synchron zum Stator mit. Es ist selbsterklärend, dass diese Reluktanzkräfte umso grösser sind, je grösser die magnetische Durchflutung ist. Die Reluktanzkräfte wirken aber nicht nur stabilisierend hinsichtlich einer Verdrehung des Rotors 2 relativ zum Lagerstator 1, sie bewirken auch eine Stabilisierung des Rotors in axialer Richtung bzw. hinsichtlich einer möglichen Verkippung des Rotors um die beiden Verkippungsachsen. Während also eine aktive Stabilisierung von zwei Freiheitsgraden (nämlich die Stabilisierung hinsichtlich einer Verschiebung des Rotors in den zwei Richtungen in der Lagerebene) erfolgt, erfolgt eine passive Stabilisierung der anderen vier Freiheitsgrade (nämlich die Stabilisierung des Rotors bezüglich der zwei Verkippungsachsen, die axiale Stabilisierung des Rotors, und die Stabilisierung gegen eine Verdrehung des Rotors relativ zum Stator in der Lagerebene) durch Reluktanzkräfte.

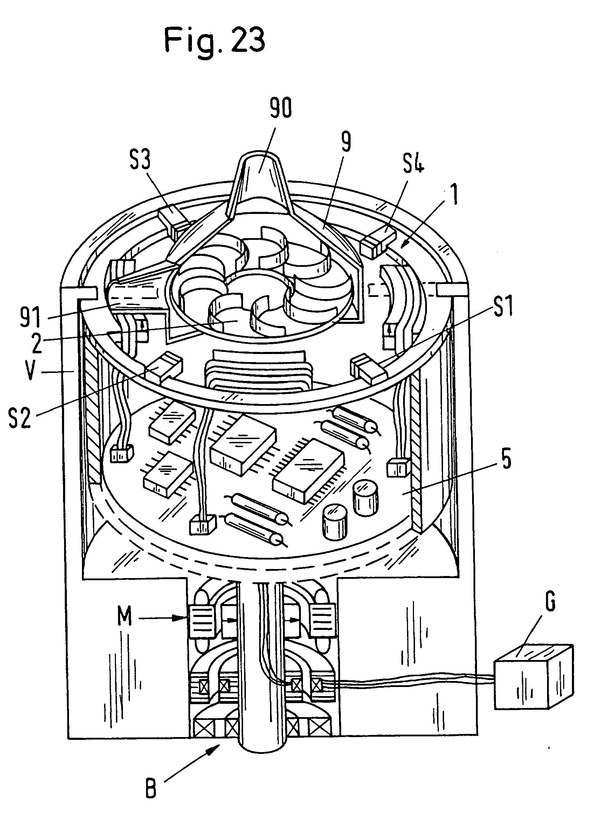

In dem in Fig. 2 gezeigten Ausführungsbeispiel der erfindungsgemässen Rotationsanordnung erkennt man wieder den Lagerstator 1 und den Rotor 2, sowie ein zwischen dem Lagerstator 1 und dem Rotor 2 angeordnetes, z.B. aus Kunststoff oder auch aus Aluminium, allgemein ein aus einem nicht ferromagnetischen Material bestehendes Behältnis 3, in dessen Innenraum der Rotor 2 angeordnet ist. Der Lagerstator 1 seinerseits ist mit einem Gestänge 4 verbunden, welches ebenfalls aus einem nicht ferromagnetischen Material, z.B. aus Kunststoff, bestehen kann (aber auch aus einem magnetisch schlecht leitfähigen Metall bestehen kann). Das Gestänge 4 ist in zwei Lagern B drehbar gelagert und kann mittels eines Motors M rotatorisch angetrieben werden. Ferner erkennt man noch schematisch eine Ansteuerelektronik 5. Diese wird mittels einer elektrischen Energiequelle G über Schleifringe S (also durch mechanischen Kontakt) und (nicht dargestellte Zuleitungen) mit elektrischer Energie versorgt, alternativ ist auch eine berührungslose induktive Energieübertragung möglich (siehe z.B. Fig. 23).

Wird nun mittels des Motors M das Gestänge 4 rotatorisch angetrieben, so wird dadurch auch der Lagerstator 1 gedreht. Der Rotor 2 folgt - wie bereits erläutert - dieser Drehbewegung des Lagerstators 1 aufgrund der Reluktanzkräfte. Auf diese Weise kann beispielsweise ein Rührwerk, eine Pumpe, insbesondere ein Pumpe für hochreine oder biologische Flüssigkeiten, speziell für Blut, angetrieben werden. Andere Anwendungen einer derartigen Rotationsanordnung sind z.B. ein Gebläse für Reinraumanwendungen, oder eine Trägervorrichtung für Wafer. Hierauf wird weiter unten noch eingegangen.

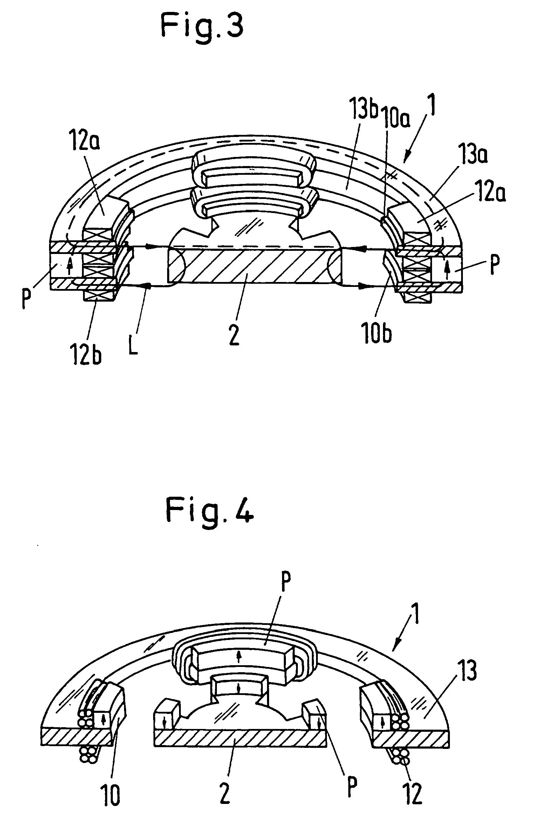

Fig. 3 zeigt ein Ausführungsbeispiel des Lagerstators 1 und des Rotors 2 in einer vegrösserten Darstellung. Man erkennt, dass der Lagerstator 1 hier zwei Statorringe 13a und 13b umfasst, von denen die Zähne 10a und 10b jeweils nach innen in Richtung des Rotors 2 abstehen. Zwischen den beiden aus ferromagnetischem Material bestehenden Statorringen 13a und 13b (z.B. aus Eisen) sind in axialer Richtung magnetisierte Permanentmagnete P angeordnet. Ferner erkennt man die um die jeweiligen Zähne 10a bzw. 10b herum gewickelten Wicklungen 12a bzw. 12b, die hier als reine Steuerwicklungen ausgebildet sind, um zu jeder Zeit das magnetische Kräftegleichgewicht zu gewährleisten.

Der von den Permanentmagneten P erzeugte magnetische Fluss verläuft vom Pemanentmagneten P ausgehend durch den Zahn 10a des oberen Statorrings 13a, über den Luftspalt zwischen Lagerstator 1 und Rotor 2, durch den Rotor 2, wieder über den Luftspalt zurück über den Zahn 10b des unteren Statorrings 13b zum Permanentmagneten P, sodass der magnetische Kreis L geschlossen ist. Wird nun der Rotor 2 radial (also in der Lagerebene) aus seiner Sollage ausgelenkt, so kann mit Hilfe der Steuerwicklungen 12a und 12b eine Steuerdurchflutung bzw. Steuerfluss CF erzeugt werden, mit dessen Hilfe der Rotor 2 wieder in seine Sollage zurück bewegt werden kann. Der Verlauf des Steuerflusses CF ist im oberen Statorring 13a durch eine gestrichelte Linie angedeutet (analoge Betrachtungen gelten für den unteren Statorring 13b). Der Steuerfluss CF verläuft in der Ebene des Statorrings 13a. Im Bereich der Zähne überlagert er sich dem von den Permanentmagneten P erzeugten permanentmagnetischen Fluss, sodass dieser entweder verstärkt oder geschwächt werden kann, je nachdem, in welcher Richtung der Rotor 2 bewegt werden muss, um in seine Sollage zurückzukehren. Bei diesem Ausführungsbeispiel ist der Rotor 2 aus einem ferromagnetischen Werkstoff, z.B. aus Eisen, hergestellt, weist aber keinerlei Besonderheiten auf, sodass er kostengünstig hergestellt werden kann. Dies ist insbesondere von Vorteil, wenn der Rotor 2 aufgrund seines Einsatzgebiets als Wegwerfteil ausgebildet sein muss bzw. nach der Anwendung rezykliert werden muss. Darüberhinaus wird bei diesem Ausführungsbeispiel die Lagerkraft in der Sollage des Rotors 2 praktisch vollständig von den Permanentmagneten P aufgebracht (magnetische Vorspannung), wodurch einerseits der elektrische Energieverbrauch gering gehalten wird (und somit auch nur geringe Verlustwärme anfällt), gleichzeitig aber eine hohe Durchflutung und damit auch grosse Reluktanzkräfte erzeugt werden.

Das in Fig. 4 gezeigte Ausführungsbeispiel des Lagerstators 1 und des Rotors 2 unterscheidet sich von dem in Fig. 3 gezeigten Ausführungsbeispiel im wesentlichen dadurch, dass bei dem in Fig. 4 gezeigten Ausführungsbeispiel nur ein Statorring 13 vorgesehen ist, und dadurch, dass nicht nur im Lagerstator 1, sondern auch am Rotor 2 in axialer Richtung magnetisierte Permanentmagnete P vorgesehen sind. Da der zu überbrückende Luftspalt bei dem Ausführungsbeispiel gemäss Fig. 4 grösser ist als bei dem Ausführungsbeispiel gemäss Fig. 3 (der Rückschluss für den permanentmagnetisch erregten Fluss erfolgt ja über die Luft, wobei der Fluss zunächst in axialer Richtung gerichtet ist), sind auch am Rotor 2 Permanentmagnete P vorgesehen. Diese Anordnung ist daher insofern ein wenig aufwendiger, als am Rotor 2 ebenfalls Permanentmagnete 2 vorgesehen sind. Dafür ist die Anordnung hinsichtlich ihrer axialen Abmessungen noch kleiner als bei dem Ausführungsbeispiel gemäss Fig. 3. Ausserdem können Verkippungen des Rotors 2 noch besser stabilisiert werden.

Es ist selbstverständlich, dass auch bei dem Ausführungsbeispiel gemäss Fig. 3 am Rotor 2 Permanentmagnete P vorgesehen sein können, z.B. kann der Rotor 2 zwei ferromagnetische Scheiben umfassen, zwischen denen entsprechend magnetisierterte Permanentmagnete P angeordnet sein können. Dadurch werden die Reluktanzkräfte noch weiter erhöht bzw. es kann ein grösserer Luftspalt zwischen Lagerstator 1 und Rotor 2 toleriert werden, ohne dass die Funktionstüchtigkeit darunter leidet. Allerdings ist der Rotor 2 - wegen der zusätzlichen Permanentmagnete P - ein wenig aufwendiger. Alternativ könnte die Dimensionierung gleich bleiben wie in Fig. 3 gezeigt, dann hat die Ausstattung des Rotors 2 mit Permanentmagneten P zur Folge, dass auf diese Weise noch grössere Reluktanzkräfte bewirkt werden können.

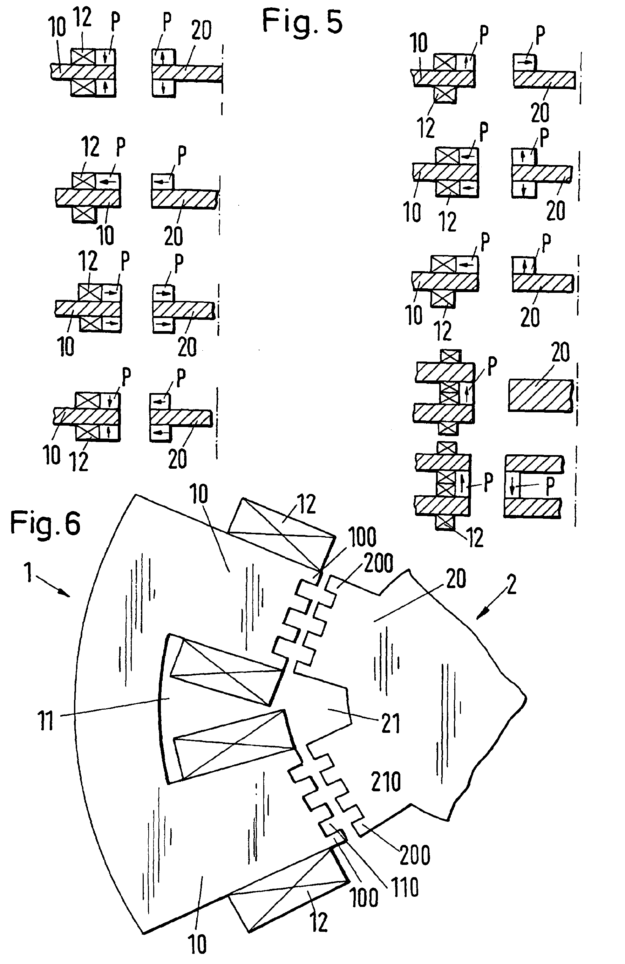

Weitere mögliche Beispiele von Anordnungen von Permanentmagneten P auf dem Lagerstator 1 und dem Rotor 2, insbesondere in radialer Richtung magnetisierte Permanentmagnete P sowie Kombinationen von axial magnetisierten und radial magnetisierten Permanentmagneten P, sind in Fig. 5 dargestellt. Es sind dies, der Reihenfolge nach zunächst in der linken Spalte von oben nach unten und anschliessend in der rechten Spalte:
i) Beidseitig des Rotors 2 (bei den Zähnen 20) und entsprechend im Stator 1 (bei den Zähnen 10) angeordnete axial magnetisierte Permanentmagnete P;
ii) einseitig des Rotors 2 (z.B. oben auf dem Rotor) und entsprechend im Stator 1 angeordnete radial magnetisierte Permanentmagnete P;
iii) beidseitig des Rotors 2 und entsprechend im Stator 1 angeordnete radial magnetisierte Permanentmagnete P;
iv) beidseitig des Rotors 2 angeordnete axial magnetisierte Permanentmagnete und gleichzeitig entsprechend im Stator 1 angeordnete radial magnetisierte Permanentmagnete P;
v) einseitig des Rotors 2 (z.B. oben auf dem Rotor) angeordnete radial magnetisierte Permanentmagnete P und entsprechend im Stator 1 angeordnete axial magnetisierte Permanentmagnete P;
vi) beidseitig des Rotors angeordnete axial magnetisierte Permanentmagnete P und entsprechend im Stator 1 angeordnete radial magnetisierte Permanentmagnete P;
vii) einseitig des Rotors 2 (z.B. auf dem Rotor) angeordnete axial magnetisierte Permanentmagnete P und entsprechend im Stator 1 angeordnete radial magnetisierte Permanentmagnete P;
viii) keine Permanentmagnete im Rotor 2, axial magnetisierte Permanentmagnete P und zwei Steuerwicklungen im Stator 1;
ix) axial magnetisierte Permanentmagnete P im Rotor 2 und im Stator 1, zwei Steuerwicklungen im Stator 1. Es ist selbsterklärend, dass die Magnetisierungsrichtungen in den einzelnen in Fig. 5 gezeigten Beispielen auch umgekehrt werden können.

In Fig. 6 erkennt man einen Ausschnitt von einander gegenüberliegenden Zähnen 10 und 20 des Lagerstators 1 und des Rotors 2. Die einander zugewandten Flächen der Zähne 10 und 20 weisen ihrerseits einander gegenüberliegende Zähne 100 und 200 und dazwischen liegende Nuten 110 und 210 auf, deren Anzahl und räumliche Anordnung übereinstimmt. Auf diese Weise können die Reluktanzkräfte noch einmal vergrössert werden, wodurch auch das maximal übertragbare Drehmoment noch einmal gesteigert wird.

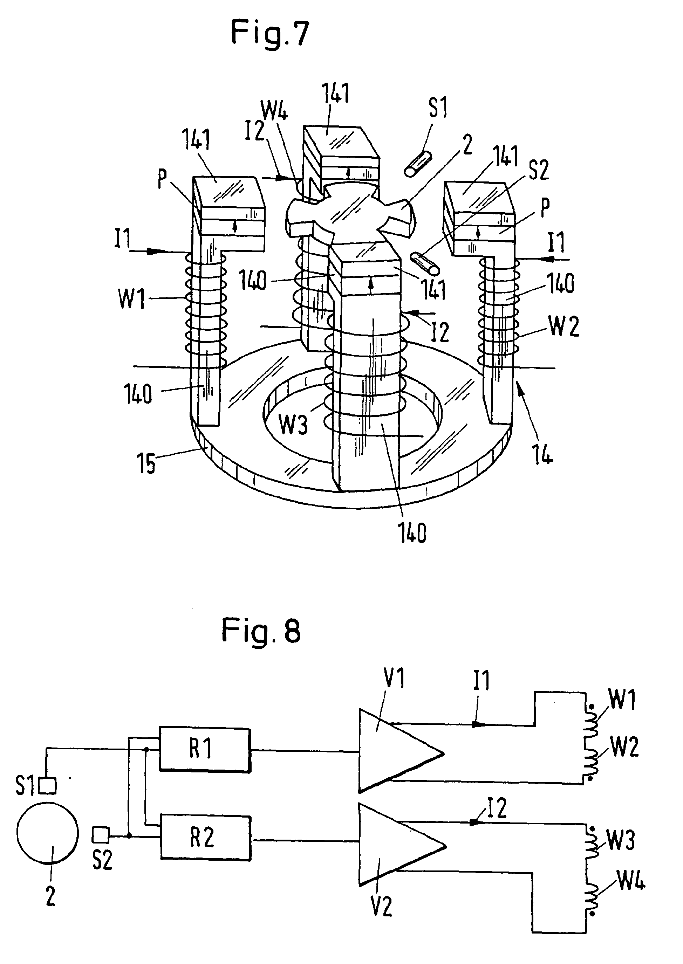

Fig. 7 zeigt ein weiteres Ausführungsbeispiel einer erfindungsgemässen Rotationsanordnung, eine sogenannte Tempelanordnung. Bei dieser Tempelanordnung umfasst der Lagerstator eine ferromagnetische Bodenscheibe bzw. einen Bodenring 15. Von diesem Bodenring 15 stehen in axialer Richtung L-förmige ferromagnetische Wicklungskerne 14 ab, deren längere Schenkel 140 in axialer Richtung weisen und dere kürzere Schenkel 141 in radialer Richtung aufeinander zu weisen und somit praktisch die Zähne des Lagerstators bilden. Um die längeren Schenkel 140 sind Steuerwicklungen 12 angeordnet, während im Bereich der kürzeren Schenkel 141 hier axial magnetisierte Permanentmagnete P vorgesehen sind, die bei Sollage des Rotors praktisch vollständig die Lagerkräfte erzeugen (magnetische Vorspannung). Einander gegenüberliegende Wicklungen W1,W2 bzw. W3,W4 weisen einen gegenläufigen Wicklungssinn auf und können in Serie geschaltet sein und ggf. von dem Steuerstrom I1 bzw. I2 durchströmt werden, um eine Abweichung des Rotors von der Sollage korrigieren zu können. Die Wicklungen W1,W2 bzw. W3,W4 können auch parallel geschaltet sein. Eine Abweichung des Rotors 2 aus seiner Sollage wird mittels der Sensoren S1,S2 festgestellt und an die bereits früher erwähnte Steuerelektronik (hier nicht dargestellt) kommuniziert. Die Steuerelektronik speist dann entsprechende Steuerströme I1,I2 in die Wicklungen W1,W2 bzw. W3,W4 ein, sodass der Rotor 2 wieder in seine Sollage zurückbewegt wird. Wegen der von den Permanentmagneten P erzeugten Durchflutung und wegen des gegenläufigen Wicklungssinns der Wicklungen W1,W2 bzw. W3,W4 wird immer gleichzeitig auf einer Seite einer Wicklung eine Erhöhung der Durchflutung und damit eine grössere Anziehungskraft auf den Rotor 2 bewirkt, während auf der anderen Seite (wegen des gegenläufigen Wicklungssinns) eine Verringerung der Durchflutung und damit der Anziehungskraft bewirkt wird. Die L-förmigen Wicklungskerne 14 sowie der Bodenring 15 können geblecht sein, also aus mehreren Lagen von Blechen bestehen, um Wirbelstromverluste zu verringern. Eine solche Tempelanordnung ist insbesondere deshalb vorteilhaft, weil der Rotor 2 auf sehr einfache Weise zugänglich ist bzw. ein Behältnis, z.B. eine Wegwerfpumpe, in deren Innenraum der Rotor angeordnet ist, auf einfache Weise zwischen die Wicklungskerne eingebracht und wieder entnommen werden kann.

Eine derartig wirkende Steuerelektronik ist in Fig. 8 schematisch dargestellt, die Wicklungen W1,W2 bzw. W3,W4 sind dabei in Serie geschaltet. Der Punkt am oberen bzw. unteren Ende der jeweiligen Wicklung W1,W2 bzw. W3,W4 steht repräsentativ für den jeweils gegenläufigen Wicklungssinn. Man erkennt, dass die von den Sensoren S1 und S2 erzeugten Eingangssignale jeweils beiden Regelkreisen R1 und R2 (z.B. PID-Reglern) zugeführt werden, da die Sensoren S1 und S2 in Fig. 7 ja so angeordnet sind, dass eine Bewegung des Rotors 2 z.B. auf den Sensor S1 zu aufgrund der Anordnung des Sensors S1 sowohl einen Steuerstrom I1 in den Wicklungen W1,W2 wie auch einen Steuerstrom I2 in den Wicklungen W3,W4 zur Folge haben muss, um den Rotor 2 in seine Sollage zurück zu bewegen. Hierzu werden die von den Reglern R1 und R2 erzeugten Signale jeweils einem (Leistungs-) Verstärker V1 bzw. V2 zugeführt, der dann den entsprechenden Steuerstrom I1 bzw. I2 in die Wicklungen W1,W2 bzw. W3,W4 einprägt.

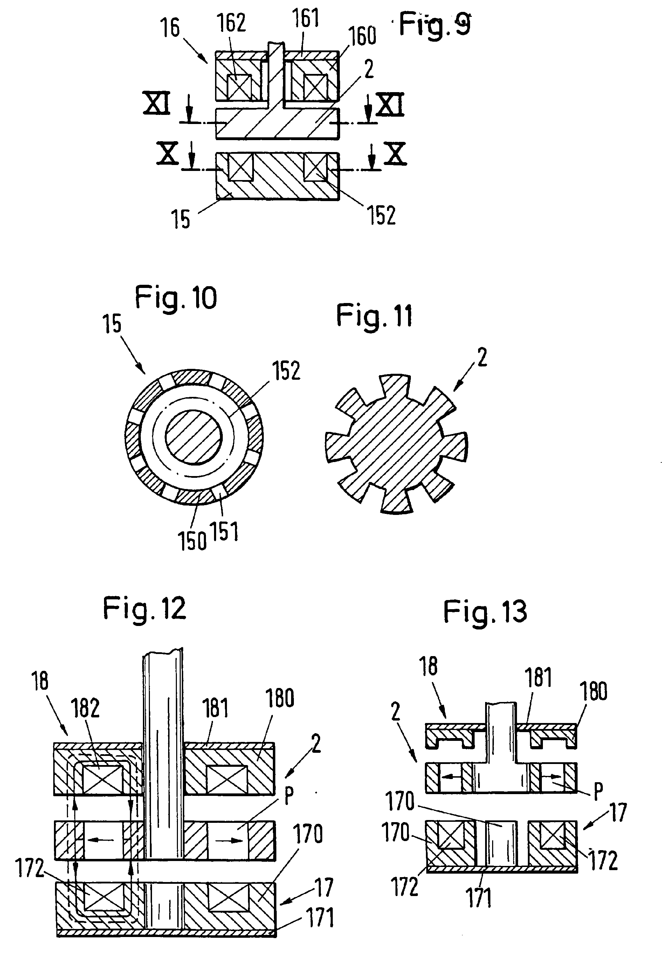

In Fig. 9 ist ein weiteres Ausführungsbeispiel der erfindungsgemässen Rotationsanordnung dargestellt, bei welchem der Lagerstator einen Topfkern 15 aufweist und einen Statorring 16. Zwischen dem Topfkern 15 und dem Statorring 16 ist der Rotor 2 angeordnet. Der Topfkern 15 weist entlang seines Umfangs Nuten 151 sowie zwischen diesen Nuten in axialer Richtung auf den Rotor 2 zuweisende Zähne 150 auf. Dies ist besser anhand des Schnitts in Fig. 10 zu erkennen, in welcher einen Schnitt entlang der Linie X-X in Fig. 9 dargestellt ist. Man erkennt, dass es sich um einen achtpoligen Topfkern 15 handelt und folgerichtig auch der Statorring 16 und der Rotor 2 achtpolig ausgebildet sein müssen. Der Rotor ist in Fig. 11 zu erkennen, welche einen Schnitt entlang der Linie XI-XI in Fig. 9 darstellt.

Der Statorring 16 weist eine Bodenscheibe bzw. einen Bodenring 161 auf mit einer zetralen Öffnung zur Durchführung des Rotorschafts, sowie von diesem Bodenring 161 in Richtung auf den Rotor 2 zuweisende U-förmige "Zähne" 160, die als U-förmige Wicklungskerne ausgebildet sind. Jeder dieser U-förmigen Zähne 160 ist mit einer eigenen Steuerwicklung 162 versehen, während in dem Topfkern 15 eine für den Topfkern 15 gesamthafte Ringwicklung 152 eingelegt ist. Während mit der Ringwicklung 152 aber lediglich immer nur eine über den gesamten Topfkern 15 gleichmässige Änderung der Durchflutung hervorgerufen werden kann, also eine Verkippung der Rotors 2 nicht korrigiert werden kann, kann eine solche Verkippung des Rotors 2 mittels der Steuerwicklungen 162 korrigiert werden, wenn beispielsweise die Steuerwicklungen von vier um 90° versetzt angeordneten Zähnen separat ansteuerbar sind.

Alternativ kann auch anstelle des Statorrings 16 ein weiterer Topfmagnet vorgesehen sein, z.B. mit einer zentralen Öffnung, durch die der Schaft des Rotors 2 geführt sein kann. In einen solchen Topfmagneten kann ebenfalls eine Ringwicklung eingelegt sein. Allerdings ist bei einer solchen Anordnung mit zwei Topfmagneten und jeweils nur einer Ringwicklung pro Topfmagnet eine Verkippung des Rotors praktisch nicht oder nur sehr schwer korrigierbar.

Fig. 12 zeigt ein weiteres Ausführungsbeispiel der erfindungsgemässen Rotationsanordnung mit zwei Statorringen bzw. einer Statorscheibe 17 und einem Statorring 18. Die Statorscheibe 17 kann ebenso gut auch eine zentrale Öffnung haben und somit als Statorring ausgebildet sein. Beide sind vierpolig ausgebildet, sie weisen also jeweils von der Bodenscheibe 171 bzw. von dem Bodenring 181 in Richtung des Rotors 2 abstehende U-förmige Zähne 170 bzw. 180 auf, die als U-förmige Wicklungskerne ausgebildet sind. Um jeden Zahn 170 bzw. 180 herum ist eine Steuerwicklung 172 bzw. 182 gewickelt. Der Rotor 2 ist sternförmig ausgebildet, ebenfalls vierpolig, und weist in jedem seiner Äste einen radial magnetisierten Permanentmagneten P auf.

Aus Fig. 12 ist gut der Verlauf der permanentmagnetischen Durchflutung (durchgezogene Linie) und der durch die Steuerströme in den Steuerwicklungen 172 bzw. 182 erzeugten Durchflutung zu erkennen. Während die permanentmagnetische Durchflutung jeweils durch den Permanentmagneten zu einem magnetischen Kreis geschlossen ist, ist dies bei der durch die Steuerwicklungen 172 bzw. 182 erzeugten (Steuer-)Durchflutung nicht der Fall, weil die Permanentmagneten P selbst eine schlechte magnetische Leitfähigkeit haben, sodass sich die von den Steuerwicklungen 172 bzw. 182 erzeugte magnetische Durchflutung jeweils über die Luftspalte und die jeweiligen Zähne 170 bzw. 180 zu einem magnetischen Kreis schliessen muss. Auch hier kann es so sein, dass die Steuerwicklungen 172 bzw. 182 von sich bezüglich des Rotors 2 gegenüberliegenden Zähnen 170 bzw. 180 in Serie oder parallel geschaltet sind, aber eine unterschiedlichen Wicklungssinn aufweisen. Jedenfalls ist mit einer solchen Anordnung auch eine Verkippung des Rotors aus der Sollage korrigierbar.

Fig. 13 stellt insofern eine Variante von Fig. 12 dar, als in Fig. 13 im Statorring 18 keine Steuerwicklungen vorgesehen sind, was im Prinzip auch nicht erforderlich ist, jedoch bei gleicher Stromstärke auch nur einen geringeren Rückstelleffekt auf den Rotor hervorruft. Insofern ist die Anordnung gemäss Fig. 13 eine "abgespeckte", daher aber auch weniger aufwendige, Version der Anordnung von Fig. 12.

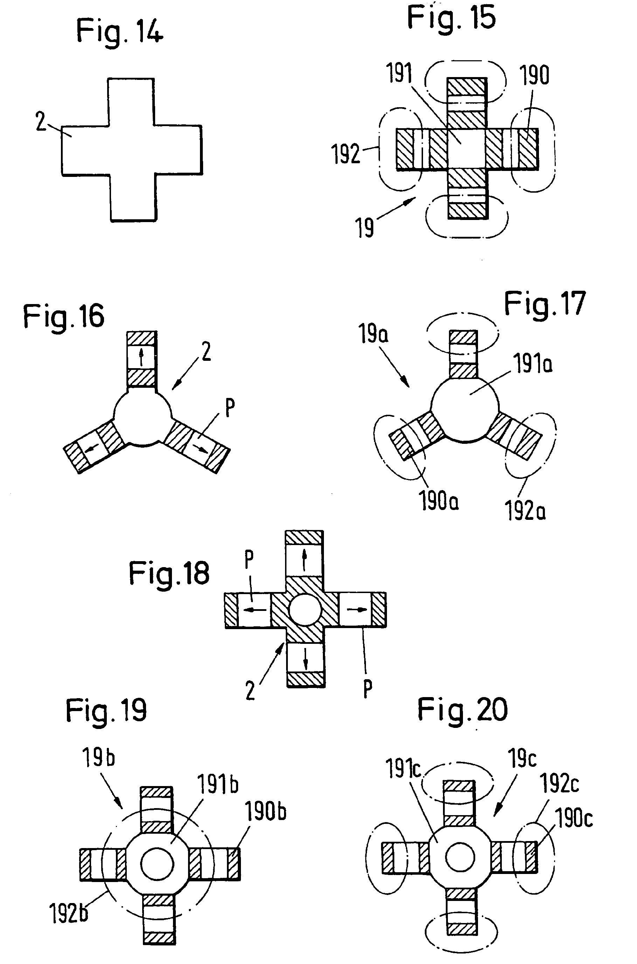

Fig. 14 zeigt ein Ausführungsbeispiel des Rotors 2, der hier sternförmig ausgebildet ist und aus einem ferromagnetischen Material hergestellt ist (und keine Permanentmagnete umfasst, ähnlich wie der Rotor in Fig. 9). Ein möglicher dazugehöriger Statorring 19 ist in Fig. 15 dargestellt. Der Statorring 19 bzw. die Statorscheibe (hier ein Statorstern) hat ein zentrales Sternteil 191 und mit diesem zentralen Sternteil 191 verbundene U-förmige Zähne 190, die als U-förmige Wicklungskerne ausgebildet sind, welche auf die Äste des sternförmigen Rotors 2 zuweisend angeordnet sind. Um jeden U-förmigen Zahn 190 ist eine separat ansteuerbare Steuerwicklung 192 gewickelt. Mit Hilfe der separat ansteuerbaren Steuerwicklungen 192 kann eine Verkippung des Rotors 2 korrigiert werden, aber es kann ausser einer Steuerdurchflutung mit Hilfe der Steuerwicklungen 192 auch zusätzlich eine Ruhedurchflutung (magnetische Vorspannung) erzeugt werden. Es ist selbsterklärend, dass ausser dem Statorring 19 noch weitere (nicht dargestellte) Mittel vorgesehen sein müssen, um die Lagerung des Rotors 2 in der Sollage erreichen zu können (es muss in der Sollage ja Kräftegleichgewicht am Rotor herrschen). Derartige Mittel sind aber hinreichend bekannt, beispielsweise kann ein gleichartiger Statorring bzw. Statorstern vorgesehen sein, der auf der anderen Seite des Rotors angeordnet ist.

Fig. 16 zeigt ein Ausführungsbeispiel des Rotors 2, der sternförmig mit einem zentralen Sternteil und mit drei gleichmässig über den Umfang verteilten Ästen ausgebildet ist. In jedem der Äste sind Permanentmagnete P angeordnet. Ein entsprechender Statorring 19a (ein dreiästiger Statorstern) ist in Fig. 17 dargestellt, und dieser weist ein zentrales Sternteil 191a und mit diesem zentralen Sternteil 191a verbundene U-förmige Zähne 190a auf. Um jeden U-förmigen Zahn 190a ist eine separat ansteuerbare Steuerwicklung 192a gewickelt, sodass eine Verkippung des Rotors 2 korrigiert werden kann. Auch hier ist klar, dass ausser der Steuerwicklung noch zusätzliche (nicht dargestellte) Mittel vorhanden sein müssen, um die Lagerung des Rotors 2 in der Sollage zu bewirken. Im Prinzip ist zur Stabilisierung einer möglichen Verkippung des Rotors lediglich ein sternförmiger Rotor mit mindestens zwei von dem zentralen Sternteil ausgehenden Ästen erforderlich, die Äste sind dabei aber in Umfangsrichtung um einen Winkel ungleich 180° gegeneinander versetzt.

Schliesslich ist in Fig. 18 ein sternförmiger Rotor 2 mit vier Ästen und in diesen Ästen angeordneten Permanentmagneten P gezeigt, die in radialer Richtung magnetisiert sind. Mögliche zugehörige Statorringe 19b bzw. 19c (vierästige Statorsterne) sind in Fig. 19 und Fig. 20 dargestellt. Während in Fig. 19 der Statorring 19b mit den Zähnen 190b eine eingelegte Ringwicklung 192b aufweist, mit welcher eine Verkippung des Rotors 2 nicht korrigiert werden kann, ist bei dem Statorring 19c in Fig. 20 um jeden Zahn 190c eine eigene, separat ansteuerbare Steuerwicklung 192c vorgesehen, mit deren Hilfe eine Verkippung des Rotors 2 korrigiert werden kann.

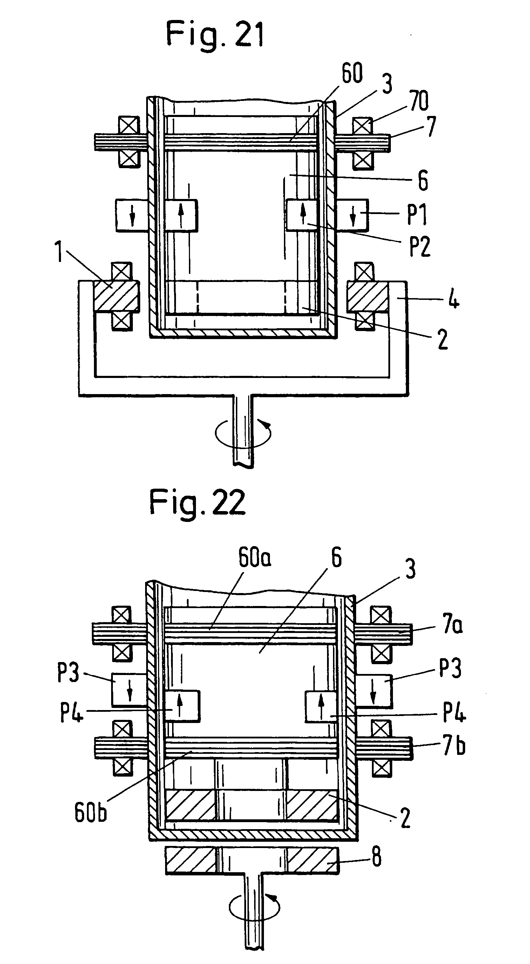

In Fig. 21 ist nun eine Anwendung einer erfindungsgemässen Rotationsanordnung dargestellt. Man erkennt hier einen Rotor 2, der mit einem Körper 6 verbunden ist. Der Rotor 2 und der mit diesem verbundene Körper 6 sind innerhalb eines Gefässes 3 angeordnet, das aus einem magnetisch schlecht leitenden Material besteht, z.B. aus Kunststoff oder Chromstahl oder einem anderen Material, welches z.B. hinsichtlich eines ebenfalls im Gefäss 3 befindlichen Mediums besonders vorteilhafte Eigenschaften aufweist.

Die magnetische Lagerung des Rotors 2 und des mit diesem Rotor 2 verbundenen Körpers 6 erfolgt über ein oberes Radialmagnetlager herkömmlicher Art, wobei auf dem Körper 6 ein geblechter ferromagnetischer Ring 60 aufgebracht ist, der zusammen mit dem ringförmigen Stator 7 mit den Statorwicklungen 70 das Radialmagnetlager bildet. Bei diesem Radialmagnetlager ist der Körper 6 in der Lagerebene frei rotierbar (die Rotation gegenüber dem Stator ist also nicht durch Reluktanzkräfte behindert), hingegen wird aber durch Reluktanzkräfte sowohl eine Verkippung des Körpers 6 als auch eine axiale Verschiebung verhindert. Zur Verhinderung einer axialen Verschiebung des Körpers 6 ist ferner ein Axialmagnetlager vorgesehen, welches zwei in axialer Richtung magnetisierte ringförmige Permanentmagnete P1 und P2 umfasst. Die Permantenmagnete P1 und P2 sind auf gleicher Höhe angeordnet. Wird nun der Körper 6 aus seiner Sollage axial ausgelenkt, so üben die Permanentmagnete P1 und P2 eine passive axiale Rückstellkraft auf den Körper 6 aus.

Schliesslich ist am unteren Ende noch eine erfindungsgemässe Rotationsanordnung mit Stator 1 und Rotor 2 vorgesehen. Hierbei handelt es sich um ein Radialmagnetlager, wie es von seinem Prinzip her in Fig.1 bzw. Fig. 2 dargestellt ist. Der Stator 1 ist mit einem Drehgestänge 4 (siehe z.B. Fig. 2) verbunden, welches motorisch antreibbar ist. Beim Antreiben des Drehgestänges 4 und damit beim Drehen des Stators 1 dreht sich der Rotor 2 - wie bereist erläutert - und damit automatisch der mit dem Rotor 2 verbundene Körper 6 mit. Im Unterschied zu dem oberen, herkömmlichen, Radialmagnetlager ist es also bei dem unteren Radialmagnetlager so, dass nicht nur eine Verkippung und eine axiale Auslenkung des Rotors 2 passiv verhindert werden bzw. Reluktanzkräfte ausgeübt werden, sondern es wird auch eine Drehung des Rotors 2 relativ zum Stator 1 durch Reluktanzkräfte verhindert.

Fig. 22 zeigt eine weitere Anwendung der erfindungsgemässen Rotationsanordnung (axiale Magnetlagerung). Der Rotor 2 ist mit dem Körper 6 verbunden und beide sind wieder innerhalb eines Gefässes 3 angeordnet, welches aus einem magnetisch schlecht leitenden Material besteht. Ferner erkennt man zwei herkömmliche Radialmagnetlager mit geblechten, ringförmigen Statoren 7a und 7b sowie mit entsprechenden auf dem Körper 6 aufgebrachten geblechten ferromagnetischen Ringen 60a und 60b. Bei diesen beiden Radialmagnetlagern ist der Körper 6 wieder in den beiden Lagerebenen frei gegenüber den Statoren drehbar, wird also nicht durch Reluktanzkräfte behindert. Hingegen wird sowohl eine axiale Auslenkung des Körpers 6 aus der Sollage wie auch eine Verkippung des Körpers 6 durch Reluktanzkräfte behindert.

Darüberhinaus ist noch eine axiale Lagerung vorgesehen. Es sind nämlich axial magnetisierte Permanentmagnete P3 und P4 vorgesehen, die in axialer Richtung gegeneinander versetzt angeordnet sind. Der in Fig. 22 gezeigte Versatz der Permanentmagnete P3 und P4 bewirkt nämlich, dass auf den Körper 6 mitsamt dem Rotor 2 eine nach oben gerichtete Kraft wirkt, welcher allerdings eine nach unten wirkende Kraft zwischen einer Statorscheibe 8 und dem Rotor 2 entgegenwirkt, sodass in der Sollage Kräftegleichgewicht herrscht. Eine Drehung der Statorscheibe 8 bewirkt über Reluktanzkräfte eine Drehung des Rotors 2 und damit des Körpers 6, der sich ja in der Lagerebene der beiden Radialmagnetlager ungehindert drehen kann.

Fig. 23 schliesslich zeigt ein Ausführungbeispiel einer Anwendung der erfindungsgemässen Rotationsanordnung in Form einer Pumpe, insbesondere einer Pumpe für hochreine oder biologische Flüssigkeiten, insbesondere eine Blutpumpe. Das Pumpengehäuse 9 kann aus einem für den Transport von Blut geeigneten Material, z.B. aus Kunststoff, bestehen. In dem Pumpengehäuse 9 ist der Rotor 2 angeordnet, der hier die Gestalt eines Flügelrads aufweist (es sind aus zeichnerischen Gründen keine Zähne des Rotors dargestellt). Das Pumpengehäuse weist eine Einlassöffnung 90 und eine Auslassöffnung 91 auf, an welche jeweils eine Zuführleitung und eine Abführleitung angeschlossen werden können. Da bei Blut als zu transportierender Flüssigkeit eine Wiederverwendung der mit Blut kontaktierten Teile aus Sicherheitsgründen nicht in Frage kommt, müssen also praktisch die mit Blut kontaktierten Teile anzahlmässig gering gehalten werden und nach Möglichkeit auch wenig aufwendig in der Herstellung sein. Wie man aus Fig. 23 erkennen kann, reduzieren sich die mit Blut kontaktierten Teile hier auf das Pumpengehäuse 9 (Kunststoffteil) und den in diesem Pumpengehäuse 9 angeordneten Rotor 2. Beim Ersetzen eines Pumpengehäuses 9 werden einfach die Zuführleitung und die Abführleitung abgezogen und anschliessend auf ein neues Pumpengehäuse aufgesteckt. Dieses Pumpengehäuse 9 wird dann in die Vorrichtung V eingesetzt. Man erkennt, dass sämtliche anderen Teile der Vorrichtung V wiederverwendet werden können, weshalb diese Lösung besonders kostengünstig ist.

Die Energieversorgung G, welche die elektrische Energie zur Versorgung der Steuerelektronik 5 bereitstellt, überträgt diese Energie hier induktiv. Ferner erkennt man antriebsseitig noch den Motor M und ein Lager B. Die Steuerlektronik 5 dreht sich bei diesem Ausführungsbeispiel mit dem Lagerstator mit. Dadurch wird vermieden, dass eine grosse Anzahl von elektrischen Verbindungen von rotierenden zu ortsfesten Teilen benötigt wird. Die jeweilige Position des Rotors 2 wird mit Hilfe von Sensoren S1,S2,S3,S4 gemessen, an die Steuerelektronik 5 kommuniziert, die nötigenfalls entsprechende Steuerströme in die Steuerwicklungen einprägt.

Ein Einsatzgebiet für die erfindungsgemässe Rotationsanordnung, nämlich eine Fördervorrichtung oder Pumpe für hochreine oder biologische Flüssigkeiten, insbesondere für Blut, ist vorstehend bereits erläutert. Die erfindungsgemässe Rotationsanordnung lässt sich aber auch sehr gut in Geräten für Reinraumanwendungen, so z.B. für Gebläse für Reinraumanwendungen oder für Wafer-Träger einsetzen, wo es darauf ankommt, dass keinerlei Verunreinigungen oder Gase von Schmiermitteln von mechanischen Lagern in den Reinraum gelangen können. Durch die magnetische Lagerung wird genau dies erreicht. Aber auch z.B. für Rührwerke sind die beschriebenen magnetgelagerten Rotationsanordnungen geeignet.

Ferner soll noch erwähnt werden, dass bei der erfindungsgemässen Rotationsanordnung, insbesondere hinsichtlich ihrer Verwendung als Blutpumpe, etliche Aspekte aus der WO-A-96/31934 ebenfalls denkbar sind. So ist sowohl eine Innenläufer- wie auch eine Aussenläuferanordnung denkbar. Weiterhin kann, wie bereits erwähnt, zwischen dem Stator und dem Rotor eine hermetische Trennstelle (wie z.B. ein Spaltrohr oder ein Gefäss) angeordnet sein. Der Rotor selbst kann mit einem Kunststoff, mit einer Keramik oder mit einem anderen nicht metallischen Werkstoff eingekapselt werden. Ferner kann ein um den Rotor herum angeordneter Ring aus elektrisch gut leitfähigem aber magnetisch schlecht leitfähigem Material vorgesehen sein, der als Sensorring für die Positionsmessung mittels Wirbelstromsensoren dienen kann. Die Bestimmung der jeweiligen Winkelposition des Rotors kann mittels Hallsensoren erfolgen, die ebenfalls als Positionssensoren dienen können, was vorteilhaft bei der Messung durch ein elektrisches leitfähiges Spaltrohr hindurch ist. Der Rotor kann zusammen mit dem ihn umgebenden Gefäss (siehe z.B. die anhand von Fig. 23 beschriebene Blutpumpe) austauschbar und wegwerfbar bzw. rezyklierbar sein, um die Sterilität garantieren zu können. Näheres zu all diesen Dingen ist der WO-A-31394 zu entnehmen.

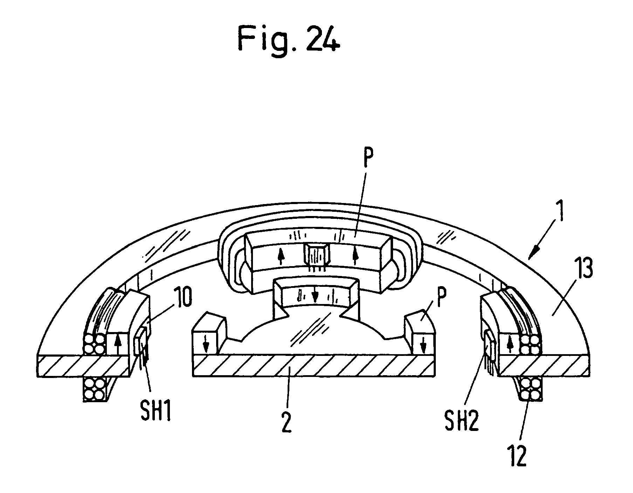

Insbesondere auf die Sensorik bei Anwendungen mit einem zwischen Stator 1 und Rotor 2 angeordneten Gefäss 3 (z.B. Fig. 21) soll aber noch einmal eingegangen werden, weil bei der Verwendung bestimmter Materialien (z.B. Chromstahl) für das Gefäss 3 die sonst typischerweise für die Bestimmung der Position des Rotors 2 eingesetzten Wirbelstromsensoren relativ schnell an ihre Grenzen stossen. Hier erweisen sich magnetische Sensoren, insbesondere die bereits erwähnten Hall-Sensoren, als besonders vorteilhaft. Wie solche Hall-Sensoren angeordnet sein können, ist in dem Ausführungsbeispiel gemäss Fig. 24 gezeigt. Das dort dargestellte Ausführungsbeispiel entspricht dem Ausführungsbeispiel gemäss Fig. 4, allerdings ergänzt um die Hall-Sensoren.

Die Hall-Sensoren können - wie anhand der Sensoren SH1 und SH2 gezeigt - im Luftspalt zwischen Stator 1 und Rotor 2 angeordnet sein, sie können insbesondere auch auf den Zähnen 10 des Stators 1 befestigt (z.B. aufgeklebt) sein. Entsprechende Sensoren sind dann auch auf den beiden anderen Zähnen vorgesehen. Die Differenz der Signale von zwei geometrisch gegenüberliegenden Sensoren eignet sich dabei besonders gut zur Bestimmung der Position des Rotors 2 in der jeweiligen Richtung, auch wenn vom Prinzip her ein Sensor je Richtung ausreicht. Alternativ können die Hall-Sensoren - wie anhand des Sensors SH3 beispielhaft gezeigt - auch auf den Permanentmagneten P aufgebracht werden, und zwar sowohl auf den Permanentmagneten P des Stators 1 wie auch auf denen des Rotors 2, da sich Stator 1 und Rotor 2 synchron drehen.

## Patentansprüche

1. Magnetgelagerte Rotationsanordnung mit einem Lagerstator (1) und einem Rotor (2), wobei der Lagerstator (1) magnetisch mit dem Rotor (2) gekoppelt ist und eine magnetische Lagerung des Rotors (2) bewirkt, wobei der Lagerstator (1) drehbar gelagert ist und ein dem Lagerstator (1) zugeordneter Drehantrieb (M) zum Drehen des Lagerstators vorgesehen ist, wobei eine Drehung des Lagerstators (1) über die magnetische Kopplung des Stators (1) mit dem Rotor (2) eine Drehung des Rotors (2) bewirkt, wobei eine dem Lagerstator zugeordnete Steuerelektronik (5) vorgesehen ist, **dadurch gekennzeichnet, dass** diese Steuerelektronik (5) zusammen mit dem Lagerstator drehbar angeordnet ist.

2. Rotationsanordnung nach Anspruch 1, **dadurch gekennzeichnet, dass** im Lagerstator (1) und im Rotor (2) Mittel vorgesehen sind, welche beim Drehen des Stators zwischen dem Stator und dem Rotor wirkende Reluktanzkräfte hervorrufen, welche eine synchrone Drehung des Rotors (2) mit dem Stator (1) zur Folge haben.

3. Rotationsanordnung nach Anspruch 1 oder 2, **dadurch gekennzeichnet, dass** die magnetische Lagerung des Rotors (2) als Radialmagnetlager ausgebildet ist, bei welchem zwei Freiheitsgrade, nämlich die Verschiebung des Rotors (2) in die zwei Richtungen der Lagerebene, aktiv regelbar sind, während die übrigen vier Freiheitsgrade, nämlich die axiale Verschiebung des Rotors, die Verdrehung des Rotors gegenüber dem Lagerstator, sowie die Verkippung des Rotors um die beiden Verkippungsachsen, über Reluktanzkräfte stabilisiert sind.

4. Rotationsanordnung nach einem der Ansprüche 1 bis 3, **dadurch gekennzeichnet, dass** sowohl der Lagerstator (1) als auch der Rotor (2) aufeinander zu weisend angeordnete Zähne (10,20) und zwischen den Zähnen des Lagerstators (1) bzw. des Rotors (2) angeordnete Nuten (11,21) aufweisen, wobei Anzahl und räumliche Anordnung der Zähne (10,20) und Nuten (11,21) von Lagerstator (1) und Rotor (2) übereinstimmen.

5. Rotationsanordnung nach Anspruch 4, **dadurch gekennzeichnet, dass** die einzelnen aufeinander zu weisend angeordneten Zähne (10,20) von Lagerstator (1) und Rotor (2) auf ihrer aufeinander zu weisenden Fläche abwechselnd Nuten (110,210) und Zähne (100,200) aufweisen, wobei die räumliche Anordnung der Nuten (110,210) und Zähne (100,200) auf den aufeinander zu weisenden Flächen der einzelnen Zähne (10,20) von Lagerstator (1) und Rotor (2) übereinstimmen.

6. Rotationsanordnung nach einem der vorangehenden Ansprüche, **dadurch gekennzeichnet, dass** im Lagerstator (1) und/oder am Rotor (2) Permanentmagnete (P) zur Erzeugung einer Vormagnetisierung vorgesehen sind.

7. Rotationsanordnung nach Anspruch 6, **dadurch gekennzeichnet, dass** nur im Lagerstator (1), nicht jedoch am Rotor (2), Permanentmagnete (P) zur Erzeugung einer Vormagnetisierung vorgesehen sind.

8. Rotationsanordnung nach Anspruch 6, **dadurch gekennzeichnet, dass** beidseitig des Rotors (2) und im Lagerstator (1) Permanentmagnete (P) vorgesehen sind.

9. Rotationsanordnung nach einem der Ansprüche 6 bis 8, **dadurch gekennzeichnet, dass** die Permanentmagnete (P) in axialer oder in radialer Richtung magnetisiert sind.

10. Rotationsanordnung nach einem der Ansprüche 4 bis 9, **dadurch gekennzeichnet, dass** im Lagerstator (1) Steuerwicklungen (12) vorgesehen sind, die den einzelnen Zähnen (10) des Lagerstators (1) zugeordnet sind.

11. Rotationsanordnung nach Anspruch 10, **dadurch gekennzeichnet, dass** der Lagerstator eine Bodenscheibe oder einen Bodenring (15) und von dieser Bodenscheibe bzw. diesem Bodenring (15) in axialer Richtung abstehende L-förmige Wicklungskerne (14) aufweist, wobei die kürzeren Schenkel (141) der L-förmigen Wicklungskerne der Bodenscheibe oder dem Bodenring (15) abgewandt angeordnet sind, aufeinander zu weisen und somit die Zähne des Lagerstators bilden, zwischen denen der Rotor (2) angeordnet ist.

12. Rotationsanordnung nach Anspruch 11, **dadurch gekennzeichnet, dass** die im Lagerstator vorgesehenen Steuerwicklungen (W1,W2,W3,W4) um die längeren Schenkel (140) der L-förmigen Wicklungskerne (14) herum angeordnet sind.

13. Rotationsanordnung nach Anspruch 1 oder 2, **dadurch gekennzeichnet, dass** die magnetische Lagerung des Rotors (2) als Axialmagnetlager ausgebildet ist, bei welchem drei Freiheitsgrade aktiv regelbar sind, nämlich die axiale Verschiebung des Rotors (2) und die Verkippung des Rotors (2) um die beiden Verkippungsachsen, während die übrigen drei Freiheitsgrade, nämlich die Verschiebung des Rotors in die zwei Richtungen der Lagerebene, sowie die Verdrehung des Rotors gegenüber dem Lagerstator, über Reluktanzkräfte stabilisiert sind.

14. Rotationsanordnung nach Anspruch 13, **dadurch gekennzeichnet, dass** der Rotor (2) eine im wesentlichen sternförmige Gestalt mit mindestens zwei von einem zentralen Sternteil ausgehenden Ästen aufweist, vorzugsweise mindestens drei Äste, die in Umfangsrichtung gleichmässig verteilt angeordnet sind, wobei in den einzelnen Ästen in radialer Richtung magnetisierte Permanentmagnete (P) vorgesehen sind.

15. Rotationsanordnung nach Anspruch 13, **dadurch gekennzeichnet, dass** der Lagerstator zwei Statorringe (18) bzw. Statorscheiben (17) aufweist, von denen jeweils der eine (17) auf der einen Seite und der andere (18) auf der anderen Seite des Rotors (2) angeordnet ist, wobei jeder Statorring (18) bzw. Statorscheibe (17) auf die Äste des sternförmigen Rotors (2) zuweisend angeordnete U-förmige Zähne (170,180) aufweist, in welche Steuerwicklungen (172,182) eingelegt sind.

16. Rotationsanordnung nach Anspruch 15, **dadurch gekennzeichnet, dass** nur in die U-förmigen Zähne (170) von einem der beiden Statorringe bzw. Statorscheiben (17) jeweils eine separat ansteuerbare Steuerwicklung (172) eingelegt ist, während in die U-förmigen Zähne (180) des anderen Statorrings (18) bzw. Statorscheibe keine Wicklungen eingelegt sind oder eine Ringwicklung eingelegt ist.

17. Rotationsanordnung nach Anspruch 13, **dadurch gekennzeichnet, dass** der Rotor (2) sternförmig mit genau drei Ästen ausgebildet ist, und dass der Lagerstator einen Statorstern (19a) mit ebenfalls genau drei Ästen aufweist, wobei der Statorstern (19a) auf die Äste des sternförmigen Rotors (2) zuweisend angeordnete U-förmige Zähne (190a) aufweist, in welche jeweils eine separat ansteuerbare Steuerwicklung (192a) eingelegt ist.

18. Rotationsanordnung nach Anspruch 13, **dadurch gekennzeichnet, dass** der Rotor (2) sternförmig mit genau vier Ästen ausgebildet ist, und dass der Lagerstator einen Statorstern (19b) mit genau vier Ästen aufweist, wobei der Statorstern (19b) auf die Äste des sternförmigen Rotors (2) zuweisend angeordnete U-förmige Zähne (190b) aufweist.

19. Rotationsanordnung nach Anspruch 16, **dadurch gekennzeichnet, dass** in die einzelnen U-förmigen Zähne (190b) des Statorsterns (19b) jeweils eine separat ansteuerbare Steuerwicklung (192b) eingelegt ist.

20. Rotationsanordnung nach Anspruch 13, **dadurch gekennzeichnet, dass** der Lagerstator einen Topfkern (15) umfasst, der in radialer Richtung verlaufende Nuten (151) aufweist sowie zwischen diesen Nuten (151) in axialer Richtung auf Zähne des Rotors zu weisende Zähne (150), wobei in diesen Topfkern (15) eine Ringwicklung (152) eingelegt ist zur Erzeugung einer Vormagnetisierung, und dass der Lagerstator ferner einen auf der anderen Seite des Rotors angeordneten ringförmigen Wicklungskern (16) umfasst, der in axialer Richtung auf den Rotor (2) zu weisende, im wesentlichen U-förmige Zähne (160) aufweist, sowie in die jeweiligen U-förmigen Zähne (160) eingelegte Steuerwicklungen (162) zur Steuerung des magnetischen Flusses durch die einzelnen Zähne.

21. Fördervorrichtung, insbesondere für hochreine oder biologische Flüssigkeiten, speziell Blutpumpe, mit einer Rotationsanordnung gemäss einem der Ansprüche 1 bis 20.

22. Trägervorrichtung, insbesondere Wafer-Träger, mit einer Rotationsanordnung gemäss einem der Ansprüche 1 bis 20.

23. Gebläse, insbesondere für Reinraumanwendungen, mit einer Rotationsanordnung gemäss einem der Ansprüche 1 bis 20.

24. Rührwerk, insbesondere Spaltrohr-Rührwerk, mit einer Rotationsanordnung gemäss einem der Ansprüche 1 bis 20.

## Claims

1. A magnetically journalled rotational arrangement comprising a bearing stator (1) and a rotor (2), with the bearing stator (1) being magnetically coupled to the rotor (2) and producing a magnetic journalling of the rotor (2), wherein the bearing stator (1) is rotatably journalled and a rotary drive (M) which is associated with the bearing stator (1) is provided for the rotation of the bearing stator, with a rotation of the bearing stator (1) producing a rotation of the rotor (2) via the magnetic coupling of the stator (1) to the rotor (2), wherein an electronic control system (5) associated with the bearing stator is provided, **characterised in that** this electronic control system (5) is rotatably arranged together with the bearing stator.

2. A rotational arrangement in accordance with claim 1 **characterised in that** means are provided in the bearing stator (1) and in the rotor (2) which produce reluctance forces which act between the stator and the rotor when the stator is rotated and which result in a synchronous rotation of the rotor (2) with the stator (1).

3. A rotational arrangement in accordance with claim 1 or claim 2 **characterised in that** the magnetic journalling of the rotor (2) is designed as a radial magnetic bearing in which two degrees of freedom, namely the displacement of the rotor (2) in the two directions of the bearing plane, can be actively regulated while the remaining four degrees of freedom, namely the axial displacement of the rotor, the rotation of the rotor with respect to the bearing stator and the tilting of the rotor about the two tilt axes, are stabilised via reluctance forces.

4. A rotational arrangement in accordance with any one of the claims 1 to 3 **characterised in that** both the bearing stator (1) and the rotor (2) have teeth (10, 20) which point at one another and grooves (11, 21) which are arranged between the teeth of the bearing stator (1) and of the rotor (2) respectively, with the number and spatial arrangement of the teeth (10, 20) and the grooves (11, 21) of the bearing stator (1) and of the rotor (2) being in agreement.

5. A rotational arrangement in accordance with claim 4 **characterised in that** the individual teeth (10, 20) of the bearing stator (1) and the rotor (2) which point towards one another have alternating grooves (110, 210) and teeth (100, 200) on their surfaces which point towards one another, with the spatial arrangement of the grooves (110, 210) and the teeth (100, 200) on the surfaces which face towards one another of the individual teeth (10, 20) of the bearing stator (1) and the rotor (2) being in agreement.

6. A rotational arrangement in accordance with any one of the preceding claims **characterised in that** permanent magnets (P) for the production of a bias magnetisation are provided in the bearing stator (1) and/or at the rotor (2).

7. A rotational arrangement in accordance with claim 6 **characterised in that** permanent magnets (P) for the production of a bias magnetisation are provided only in the bearing stator (1) but not at the rotor (2).

8. A rotational arrangement in accordance with claim 6 **characterised in that** permanent magnets (P) are provided at both sides of the rotor (2) and in the bearing stator (1).

9. A rotational arrangement in accordance with any one of the claims 6 to 8 **characterised in that** the permanent magnets (P) are magnetised in the axial direction or in the radial direction.

10. A rotational arrangement in accordance with any one of the claims 4 to 9 **characterised in that** control windings (12) are provided in the bearing stator (1) which are associated with the individual teeth (10) of the bearing stator (1).

11. A rotational arrangement in accordance with claim 10 **characterised in that** the bearing stator has a base disc or a base ring (15) and L-shaped winding cores (14) projecting from this base disc or base ring (15) in the axial direction, with the shorter limbs (141) of the L-shaped winding cores being arranged to face away from the base disc or the base ring (15), pointing towards one another and thus forming the teeth of the bearing stator between which the rotor (2) is arranged.

12. A rotational arrangement in accordance with claim 11 **characterised in that** the control windings (W1, W2, W3, W4) which are provided in the bearing stator are arranged around the longer limbs (140) of the L-shaped winding cores (14).

13. A rotational arrangement in accordance with claim 1 or claim 2 **characterised in that** the magnetic journalling of the rotor (2) is formed as an axial magnetic bearing in which three degrees of freedom can be actively regulated, namely the axial displacement of the rotor (2) and the tilting of the rotor (2) about the two tilt axes, whereas the three remaining degrees of freedom, namely the displacement of the rotor in the two directions of the bearing plane and the rotation of the rotor with respect to the bearing stator, are stabilised via reluctance forces.

14. A rotational arrangement in accordance with claim 13 **characterised in that** the rotor (2) has a substantially star-shaped form with at least two branches going out from a central star part, preferably at least three branches which are arranged to be uniformly distributed in the peripheral direction, with permanent magnets (P) which are magnetised in the radial direction being provided in the individual branches.

15. A rotational arrangement in accordance with claim 13 **characterised in that** the bearing stator has two stator rings (18) or stator discs (17), of which in each case the one (17) is arranged at the one side of the rotor (2) and the other (18) is arranged at the other side, with each stator ring (18) or stator disc (17) having U-shaped teeth (170, 180) which are arranged to face towards the branches of the star-shaped rotor (2) and into which control windings (172, 182) are laid.

16. A rotational arrangement in accordance with claim 15 **characterised in that** in each case a separately excitable control winding (172) is laid into the U-shaped teeth (170) of only one of the two stator rings or stator discs (17) whereas either no windings or a ring winding are/is laid into the U-shaped teeth (180) of the other stator ring (18) or stator disc.

17. A rotational arrangement in accordance with claim 13 **characterised in that** the rotor (2) is formed in the shape of a star with exactly three branches; and **in that** the bearing stator has a stator star (19a) with likewise exactly three branches, with the stator star (19a) having U-shaped teeth (190a) which face towards the branches of the star-shaped rotor (2) and into which in each case a separately excitable control winding (192a) is laid.

18. A rotational arrangement in accordance with claim 13 **characterised in that** the rotor (2) is formed in the shape of a star with exactly four branches; and **in that** the bearing stator has a stator star (19b) with exactly four branches, with the stator star (19b) having U-shaped teeth (190b) which face towards the branches of the star-shaped rotor (2).

19. A rotational arrangement in accordance with claim 16 **characterised in that** a separately excitable control winding (192b) is laid into the individual U-shaped teeth (190b) of the stator star (19b) in each case.

20. A rotational arrangement in accordance with claim 13 **characterised in that** the bearing stator comprises a pot core (15) which has grooves (151) extending in the radial direction as well as teeth (150) between these grooves (151) which face in the axial direction towards the teeth of the rotor, with a ring winding (152) being laid into this pot core (15) for the production of a bias magnetisation; and **in that**, furthermore, the bearing stator comprises a ring-shaped winding core (16) which is arranged on the other side of the rotor and which has substantially U-shaped teeth (160) which face in the axial direction towards the rotor (2) and control windings (162) which are laid into the respective U-shaped teeth (160) for the control of the magnetic flux through the individual teeth.

21. A pumping apparatus, in particular for highly pure or biological liquids, especially a blood pump, with a rotational arrangement in accordance with any one of the claims 1 to 20.

22. A carrier apparatus, in particular a wafer carrier, comprising a rotational arrangement in accordance with any one of the claims 1 to 20.

23. A blower, in particular for clean room applications, comprising a rotational arrangement in accordance with any one of the claims 1 to 20.

24. A stirrer, in particular a canned motor stirrer, comprising a rotational arrangement in accordance with any one of the claims 1 to 20.

## Revendications

1. Agencement de rotation à palier magnétique avec un stator de palier (1) et un rotor (2), où le stator de palier (1) est couplé magnétiquement avec le rotor (2) et provoque un logement magnétique du rotor (2), où le stator de palier (1) est logé d'une manière tournante et est prévu une commande de rotation (M) associée au stator de palier (1) pour la rotation du stator de palier, où une rotation du stator de palier (1) par le couplage magnétique du stator (1) avec le rotor (2) provoque une rotation du rotor (2), où est prévue une électronique de commande (5) associée au stator de palier, **caractérisé en ce que** cette électronique de commande (5) conjointement avec le stator de palier est disposée d'une manière rotative.

2. Agencement de rotation selon la revendication 1, **caractérisé en ce que** dans le stator de palier (1) et dans le rotor (2), des moyens sont prévus qui, lors de la rotation du stator, provoquent des forces de reluctance agissant entre le stator et le rotor qui entraînent une rotation synchrone du rotor (2) avec le stator (1).

3. Agencement de rotation selon la revendication 1 ou 2, **caractérisé en ce que** le logement magnétique du rotor (2) est réalisé comme palier magnétique radial, dans lequel deux degrés de liberté, à savoir le déplacement du rotor (2) dans les deux directions du plan du palier, peuvent être réglés activement tandis que les autres quatre degrés de liberté, à savoir le déplacement axial du rotor, la rotation du rotor par rapport au stator de palier ainsi que le basculement du rotor autour des deux axes de basculement, peuvent être stabilisés par des forces de reluctance.

4. Agencement de rotation selon l'une des revendications 1 à 3, **caractérisé en ce qu'**à la fois le stator de palier (1) et aussi le rotor (2) présentent des dents (10, 20) dirigées les unes vers les autres et des rainures (11, 21) disposées entre les dents du stator de palier (1) respectivement du rotor (2), où le nombre et la disposition spatiale des dents (10, 20) et des rainures (11, 21) du stator de palier (1) et du rotor (2) coïncident.

5. Agencement de rotation selon la revendication 4, **caractérisé en ce que** les dents individuelles (10, 20) dirigées les unes vers les autres du stator de palier (1) et du rotor (2) présentent sur leur face orientée l'une vers l'autre alternativement des rainures (110, 210) et des dents (100, 200), où la disposition spatiale des rainures (110, 210) et des dents (100, 200) sur les faces orientées les unes vers les autres des dents individuelles (10, 20) du stator de palier (1) et du rotor (2) coïncident.

6. Agencement de rotation selon l'une des revendications précédentes, **caractérisé en ce que** sont prévus dans le stator de palier (1) et/ou au rotor (2) des aimants permanents (P) pour produire une pré-magnétisation.

7. Agencement de rotation selon la revendication 6, **caractérisé en ce que** sont prévus seulement dans le stator de palier (1), mais non pas au rotor (2), des aimants permanents (P) pour produire une pré-magnétisation.

8. Agencement de rotation selon la revendication 6, **caractérisé en ce que** des aimants permanents (P) sont prévus des deux côtés du rotor (2) et dans le stator de palier (1).

9. Agencement de rotation selon l'une des revendications 6 à 8, **caractérisé en ce que** les aimants permanents (P) sont aimantés dans la direction axiale ou dans la direction radiale.

10. Agencement de rotation selon l'une des revendications 4 à 9, **caractérisé en ce que** des enroulements de commande (12) sont prévus dans le stator de palier (1), qui sont associés aux dents individuelles (10) du palier de stator (1).

11. Agencement de rotation selon la revendication 10, **caractérisé en ce que** le stator de palier présente un disque de fond ou un anneau de fond (15) et des noyaux d'enroulement en forme de L (14) faisant saillie de ce disque de fond respectivement de cet anneau de fond (15) dans la direction axiale, où les branches plus courtes (141) des noyaux d'enroulement en forme de L sont détournées du disque de fond ou de l'anneau de fond (15) sont orientées les unes vers les autres et forment ainsi les dents du stator de palier entre lesquelles est disposé le rotor (2).

12. Agencement de rotation selon la revendication 11, **caractérisé en ce que** les enroulements de commande (W1, W2, W3, W4) prévus dans le stator de palier sont disposés autour des branches plus longues (140) des noyaux d'enroulement en forme de L (14).

13. Agencement de rotation selon la revendication 1 ou 2, **caractérisé en ce que** le logement magnétique du rotor (2) est réalisé comme palier magnétique axial dans lequel trois degrés de liberté peuvent être réglés d'une manière active, à savoir le déplacement axial du rotor (2) et le basculement du rotor (2) autour des deux axes de basculement, tandis que les trois autres degrés de liberté, à savoir le déplacement du rotor dans les deux directions du plan du palier ainsi que la rotation du rotor par rapport au stator de palier, peuvent être stabilisés par des forces de reluctance.

14. Agencement de rotation selon la revendication 13, **caractérisé en ce que** le rotor (2) présente une configuration essentiellement en forme d'étoile avec au moins deux branches partant de la partie d'étoile centrale, de préférence au moins trois branches qui sont réparties uniformément dans la direction du pourtour, où sont prévus dans les différentes branches des aimants permanents (P) aimantés dans la direction radiale.

15. Agencement de rotation selon la revendication 13, **caractérisé en ce que** le stator de palier présente deux anneaux de stator (18) respectivement disques de stator (17) dont à chaque fois un (17) est disposé sur un côté et l'autre (18) sur l'autre côté du rotor (2), où chaque anneau de stator (18) respectivement disque de stator (17) présente des dents en forme de U (170, 180) orientées vers les branches du rotor (2) en forme d'étoile dans lesquelles sont insérés des enroulements de commande (172, 182).

16. Agencement de rotation selon la revendication 15, **caractérisé en ce que** seulement dans les dents en forme de U (170) d'un des deux anneaux de stator respectivement disques de stator (17) est posé respectivement un enroulement de commande (172) pouvant être commandé séparément, tandis que dans les dents en forme de U (180) de l'autre anneau de stator (18) respectivement disque de stator, aucun enroulement est inséré ou bien un enroulement annulaire est inséré.

17. Agencement de rotation selon la revendication 13, **caractérisé en ce que** le rotor (2) est réalisé en forme d'étoile avec exactement trois branches, et **en ce que** le stator de palier présente une étoile de stator (19a) avec également précisément trois branches, où l'étoile de stator (19a) présente des dents en forme de U (190a) orientées vers les branches du rotor en forme d'étoile (2), dans lesquelles est placé respectivement un enroulement de commande (192a) pouvant être commandé séparément.

18. Agencement de rotation selon la revendication 13, **caractérisé en ce que** le rotor (2) est réalisé en forme d'étoile avec précisément quatre branches, et **en ce que** le stator de palier présente une étoile de stator (19b) avec exactement quatre branches, où l'étoile de stator (19b) présente des dents en forme de U (190b) orientées vers les branches du rotor en forme d'étoile (2).

19. Agencement de rotation selon la revendication 16, **caractérisé en ce qu'**est placé dans les dents individuelles en forme de U (190b) de l'étoile de stator (19b) à chaque fois un enroulement de commande (192b) pouvant être commandé séparément.

20. Agencement de rotation selon la revendication 13, **caractérisé en ce que** le stator de palier comprend un noyau en pot (15) qui présente des rainures (151) s'étendant dans la direction radiale et entre ces rainures (151) des dents (150) orientées dans la direction axiale vers les dents du rotor, où est placé dans ce noyau en pot (15) un enroulement annulaire (152) pour produire une pré-magnétisation, et **en ce que** le stator de palier comprend en outre un noyau d'enroulement annulaire (16) disposé sur l'autre côté du rotor qui présente des dents (160) orientées dans la direction axiale vers le rotor (2), sensiblement en forme de U, ainsi que des enroulements de commande (162) placés dans les dents en forme de U respectives (160) pour la commande du flux magnétique à travers les dents individuelles.

21. Dispositif de convoyage, en particulier pour des liquides très purs ou biologiques, spécialement pompe de sang, avec un agencement de rotation selon l'une des revendications 1 à 20.

22. Dispositif de support, en particulier support de plaquettes, avec un agencement de rotation selon l'une des revendications 1 à 20.

23. Soufflerie, en particulier pour des applications de salle blanche, avec un agencement de rotation selon l'une des revendications 1 à 20.

24. Agitateur, en particulier agitateur à tube à fente, avec un agencement de rotation selon l'une des revendications 1 à 20.
